# EUROPEAN PATENT SPECIFICATION

(11) **EP 3 389 006 B1**
(45) Date of publication and mention of the grant of the patent: **19.02.2020**
(21) Application number: 18161888.5
(22) Date of filing: 15.03.2018
(51) Int. Cl.: G06T 3/00

(54) **RIB UNFOLDING FROM MAGNETIC RESONANCE IMAGES**
RIPPENENTFALTUNG AUS MAGNETRESONANZBILDERN
DÉPLOIEMENT DE NERVURE À PARTIR D'IMAGES À RÉSONANCE MAGNÉTIQUE

(30) Priority: 10.04.2017 US 201715483322
(43) Date of publication of application: 17.10.2018
(73) Proprietor: Siemens Healthcare GmbH, 91052 Erlangen (DE)
(72) Inventor: Fenchel, Matthias, 91054 Erlangen (DE); Zhan, Yiqiang, West Chester, 19380 (US)

(56) References cited:
- EP-A2- 1 315 125
- US-A1- 2015 363 963
- N. RAY ET AL: "Merging parametric active contours within homogeneous image regions for MRI-based lung segmentation", IEEE TRANSACTIONS ON MEDICAL IMAGING., vol. 22, no. 2, 1 February 2003 (2003-02-01), pages 189-199, XP055487754, US ISSN: 0278-0062, DOI: 10.1109/TMI.2002.808354

## Description

### BACKGROUND OF THE INVENTION

### Field of the Invention

The present invention relates to the processing of a magnetic resonance image of the rib cage of a subject in order to show the ribs flattened or straightened into a plane.

### Description of the Prior Art

The rib cage in humans and most other mammals is a generally elliptical structure, when viewed in a cross-section orthogonal to the caudal-cranial axis, and surrounds a number of internal organs, including the lungs. In order to answer or provide insight into various types of medical questions, it is sometimes beneficial to view the ribs in a flattened or straightened form, so that the image of the ribs is basically unfolded with the ribs shown in a 2D representation, while still preserving relevant details of the individual ribs. For example, if a lesion is present on an inside surface of a rib, i.e., the surface thereof facing inwardly toward the internal organs, it may be difficult to see that lesion in a 3D image of the rib cage, even by computerized manipulation of that 3D image, because there may be other portions of the rib cage that present an obstruction. When the ribs of the rib cage are unfolded and seen flattened, however, all of their respective interior surfaces are clearly visible, and the presentation of the rib cage can be viewed with a single glance. The entire rib cage can be traversed with only a few scrolls, when all of the ribs are within a single plane.

Various image processing techniques are known that start with a volumetric (3D) data set that represents the rib cage and, by various image processing techniques, unfold the rib cage so that the ribs appear flattened or straightened. United States Patent No. 9,189,847 is an example. The most widely used of these techniques make use of a computed tomography (CT) volumetric image as the starting point, as described in United States Patent Application Publication No. 2017/0032535. This is because, being an x-ray based technique, bone structures appear with excellent contrast in a CT image, with density variations being visible.

Such a technique is also known from United States Patent Application Publication No. 2013/0101197 that starts with a nuclear medicine emission image, such as a PET (Positron Emission Tomography) or SPECT (Single Photon Emission Computed Tomography) image.

US 2015/0363963 A1 discloses a framework facilitating visualization, wherein the framework localizes at least one anatomical structure of interest in image data, which structure of interest is highlighted by reformatting the image data.

Magnetic resonance (MR) imaging is another widely used imaging modality. MR imaging has the ability to show soft tissue structures with excellent contrast, but bone structures may be less precisely visible in a conventional MR image. A desirable feature of MR imaging is that it is very sensitive to lesions of the bone marrow, and therefore is a very useful tool for the assessment of multifocal bone diseases. Another desirable feature of MR imaging is that it does not make use of x-rays, and therefore the examination subject is not exposed to x-rays in an MR scan.

It is therefore desirable, despite the less precise visual appearance of bone structures in an MR image, to be able to use or start with an MR image in order to obtain an unfolded image of the rib cage. Being able to do so would have the advantage of avoiding the exposure of the subject to x-rays and, if it happens to be the case that an MR image of a subject is the only currently available image, and at some point in the subsequent medical diagnosis a need arises to view the rib cage in planar form, it would then not be necessary to schedule another imaging session for the patient, in order to obtain a CT image to use as the starting image.

### SUMMARY OF THE INVENTION

An object of the present invention is to provide a method, non-transitory computer-readable data storage medium and an apparatus for rib unfolding, which allow the use of an MR image as the starting image that is processed in order to obtain a processed image wherein the rib cage is shown in an unfolded presentation.

This object is achieved in accordance with the present invention by a method, non-transitory computer-readable data storage medium and an apparatus wherein a computer is provided with an input data file formed of volumetric MR data that represent a 3D image of the rib cage and the lungs of a subject. In the computer, a view is selected wherein the ribs in the rib cage are represented as any smooth curved surface representing, such as a transverse slice through the 3D image, or an oblique view of the 3D image.

In the computer, the lungs in the selected view of the 3D image are used in order to define a first such smooth surface representation that is inside of the rib cage in the selected view. Also in the computer, further smooth surface representations are selectively defined starting from the first smooth curved surface representation and moving outwardly from the first smooth curved surface representation that respectively proceed through rib pairs in the rib cage in the selected image and by fitting further smooth curved surface representations to the rib cage. These further smooth curved surface representations are then used in the computer to unfold the 3D image, by cutting and straightening these smooth curved surface representations, thereby obtaining an unfolded 3D image. The unfolded 3D image is then displayed at a display in communication with the computer.

As used herein, the term "smooth curved surface representation" means a curved surface that is defined by a smooth function. A "smooth function" is a well-understood term in the field of mathematics, and has many rigorous, known mathematical definitions. The most simplistic non-mathematical definition can be considered as a curve having no corners, or that is not jagged. A commonly employed mathematical definition of a smooth curve is a curve for which all derivatives exist and are continuous. Specific examples of a smooth curved surface representation are a surface defined by an ellipse, or a B-spline, or a Bezier curve.

In the unfolded image in accordance with the invention, the unfolded ribs will not appear straight, as in a conventional unfolded image starting from a CT image, but will exhibit curves in the plane in which the ribs are unfolded. Nevertheless, all or substantially all of the interior surfaces of the ribs will be visible in the unfolded image in accordance with the invention.

In a preferred embodiment, the first smooth curved surface representation is defined by segmenting the lungs in the selected view, and defining the first smooth curved surface representation using the segmented lungs. It is also possible to define the first smooth curved surface representation manually or semi-automatically, by displaying the selected view, with a user, by user interaction with an interface such as a touchscreen, inscribing a smooth curved surface representation on the interface that proceeds through the lungs.

The identification of a smooth curved surface representation that results in the unfolding, can also be done automatically, such as by a known pattern recognition algorithm that identifies when a smooth curved surface representation, in the smooth curved surface representations proceeding outwardly from the first smooth curved surface representation, proceeds through the ribs in the selected view. It is possible in the displayed image of the selected view for a user to see when one of the successively defined smooth curved surface representations proceeds through the ribs in the displayed view to a good approximation.

### BRIEF DESCRIPTION OF THE DRAWINGS

Figure 1 schematically shows a conventional CT image of a rib cage, a conventional CT image of a rib cage, and the resulting processed image that shows the ribs in a flattened view.
Figure 2 schematically illustrates a known technique for achieving rib flattening in a processed image, using a CT image of the rib cage.
Figure 3 schematically illustrates the basis of rib flattening in an MR image in accordance with the present invention.
Figure 4 is a flowchart of an embodiment of the method according to the present invention.
Figure 5 shows an image with rib flattening that is achieved in accordance with the present invention.
Figure 6 is a block diagram of an apparatus for implementing the inventive method.

### DESCRIPTION OF THE PREFERRED EMBODIMENTS

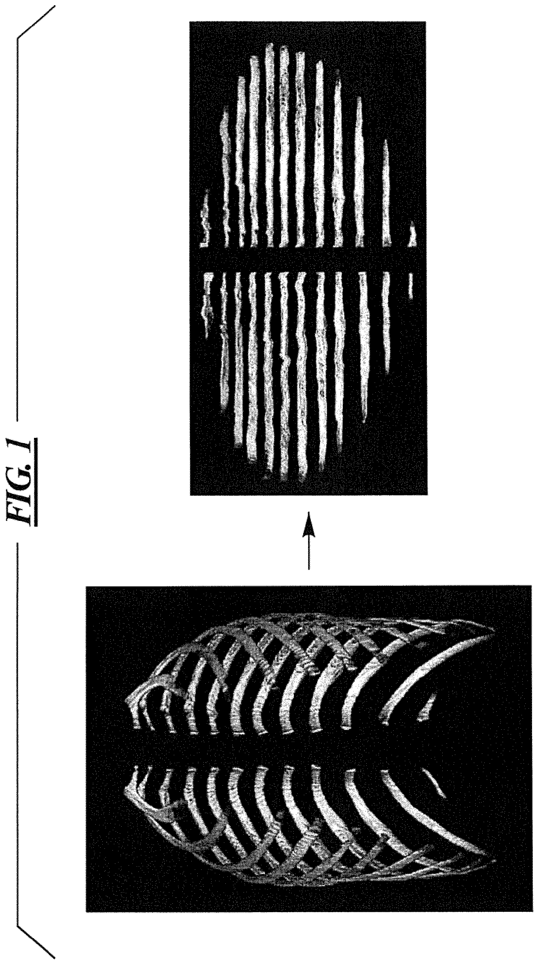

Figure 1 shows the result of a conventional procedure for rib flattening or rib straightening, starting from a conventional CT image of a rib cage, shown at the left in Figure 1. The result of the use of a conventional rib flattening algorithm is shown at the right in Figure 1, wherein the ribs are all represented in a plane, "unfolded" from the view shown at the left in Figure 1.

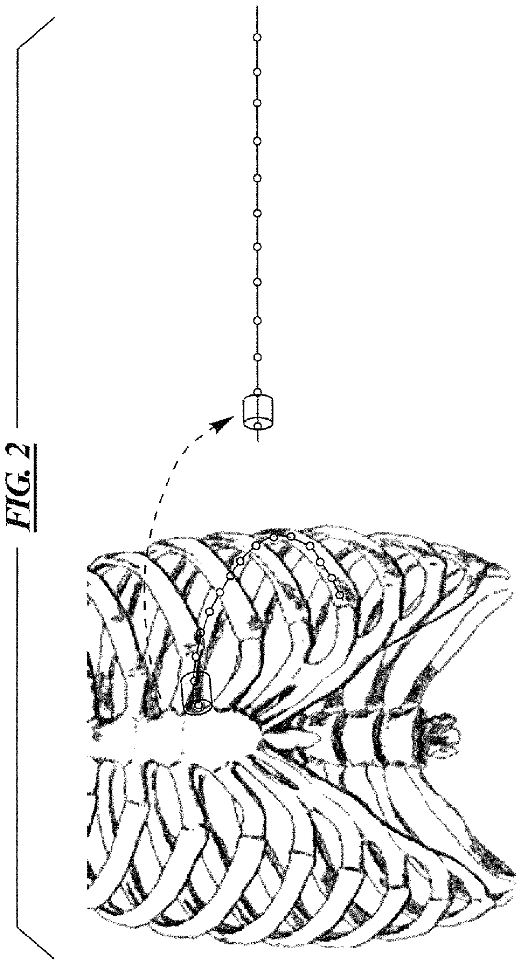

Figure 2 schematically illustrates one conventional procedure for rib flattening wherein, in an image such as shown at the left in Figure 1, each individual rib is sampled at the locations illustrated by the open circles, and a known algorithm is then employed to create a straight line from those curved samples. This known procedure requires that the ribs be clearly shown in the starting image, which is a criterion that is satisfied in a CT image, but which may not be the case in an MR image.

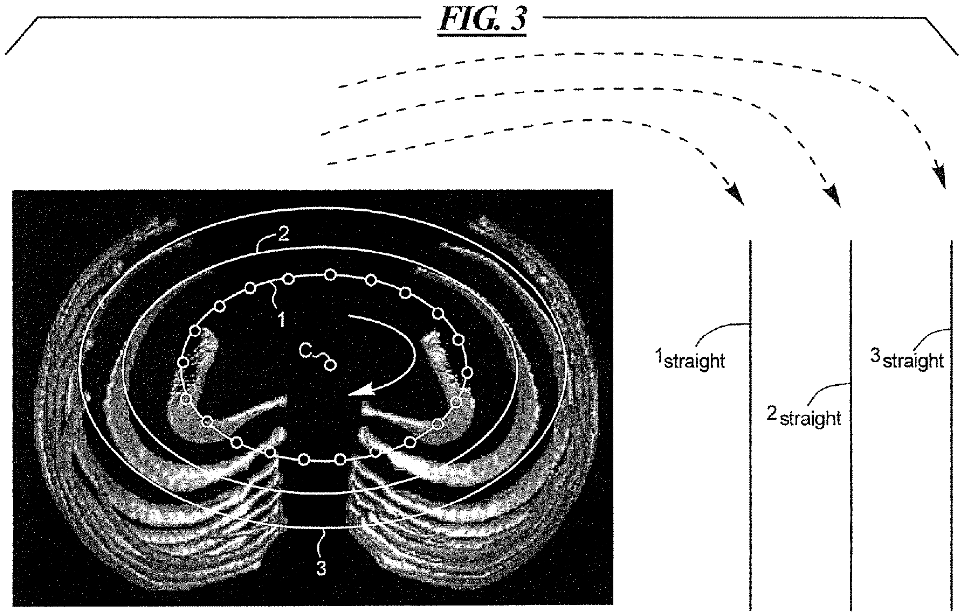

Figure 3 schematically illustrates the principles of the present invention for rib flattening starting from an MR image. A suitable MR image that can be used to implement the procedure in accordance with the invention is shown in Figure 3. This image may be a selected view of a volumetric 3D MR data set acquired from the torso of a subject, and thus shows the rib cage in its natural (curved) form, surrounding the lungs, which are also shown in Figure 3.

Using the lungs as a reference, a first smooth curved surface representation, in this embodiment indicated by the innermost ellipse with the open circles, is defined in a computer using the image shown in Figure 3. The sample points may be successively determined around the ellipse, such as by proceeding in the direction of the innermost arrow relative to a selected centerpoint C.

Starting from this innermost ellipse, further ellipses are defined that are concentric with the innermost ellipse, but that progressively proceed outwardly from the lungs, toward the rib cage.

Since the individual ribs exhibit different curvatures, the successively defined concentric ellipses will approximate the curvature of different ones, or different groups, of the ribs. In the example shown in Figure 3, the ellipse designated with reference numeral 2 approximate the ribs having the innermost curvature, and the ellipse designated with reference numeral 3 approximates the rest of the ribs. These respective ellipses are then cut in the computer, and straightened, so as to produce the straight lines designated as 1_{straight}, 2_{straight} and 3_{straight}.

Although this embodiment has been described using ellipses, any smoothed curved surface can be used, such as surface defined by a B-spline or a 3D Bezier curve.

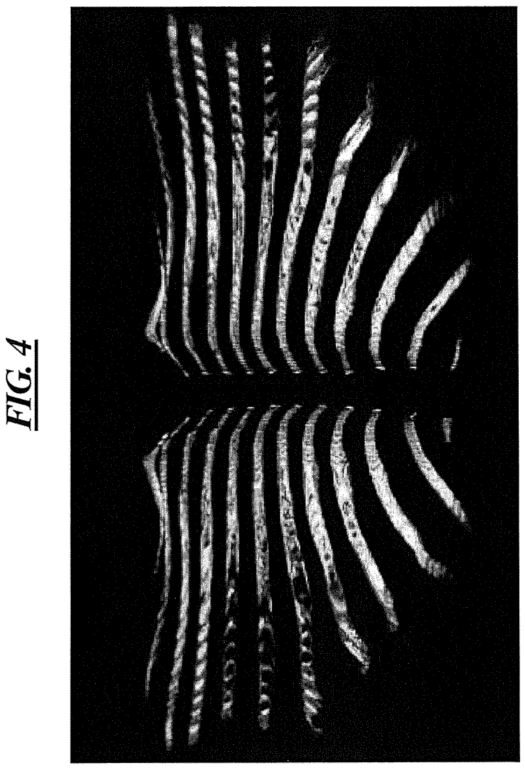

The result of the inventive procedure is shown in Figure 4. In comparison to the conventional approach shown in Figure 1, it can be seen that the ribs in the result of the inventive procedure are not completely straightened, but their interior surfaces are still visible in the flattened image that is achieved in accordance with the invention. In many types of diagnoses, it is not of significant consequence that the individual ribs are not shown as being straight since the flattened rib view is used for navigation rather than as the basic for the actual diagnosis, and so the departure from a straight rib presentation is offset by the advantage that, in accordance with the invention, only a coarse detection of the rib cage is necessary, which is a condition that can be fulfilled by most MR images of the torso. The result shown in Figure 4 still allows all ribs to be assessed in a single glance, and to be traversed with very few scroll actions.

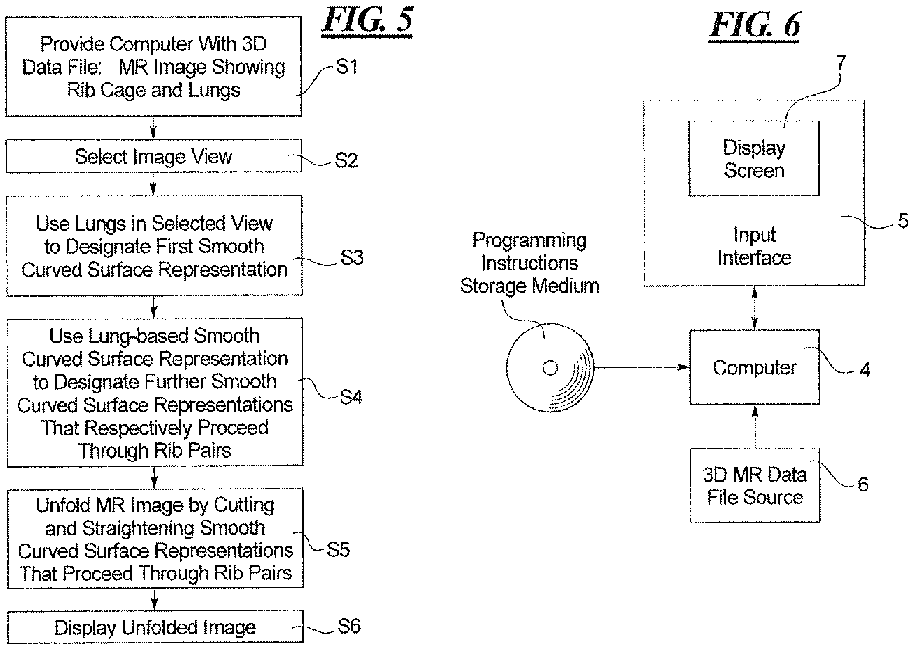

A flowchart that shows the basic steps of the method in accordance with the invention is shown in Figure 5. In a first step S1, a computer is provided with an input data file that includes volumetric MR data representing a 3D image that includes the rib cage and the lungs of a subject. This may be, for example, a volumetric MR data set of the torso of the subject.

In a second step S2, the computer selects a view through the 3D image, in which the rib cage and the lungs are visible. If it is desired to show only the lungs (and not other internal organs) in this view, these lungs can be segmented using known techniques, such as active contours, graph cuts, etc. One known procedure for lung segmentation is described in the article by Ray et al., "Merging Parametric Active Contours Within Homogeneous Image Regions for MRI-Based Lung Segmentation," IEEE Trans. on Medical Imaging, Vol. 22, No. 2, pages 189-198 (2003).

This can be followed by generating a mesh of the lung surface from the segmentation mask, such as by using the known techniques of marching cubes or marching tetrahedra. The basic marching cube or marching square technique is described in United States Patent No. 4,710,876.

The segmentation and generation of a suitable representation of the lung in the image that is used in accordance with the present invention can also be implemented manually or semi-automatically, by displaying the selected view and with a physician or technician interacting with an interface, such as a touch screen, to outline the lungs in the displayed image.

In step S3, once the lungs have been suitably designated in the image, the shape of the rib cage is predicted using the lung surfaces by fitting a smooth curved surface representation (which in turn defines a corresponding curved cylinder in the 3D data set) that best approximates the outline or shape of the lungs in the selected image. Using the lung-based smooth curved surface representation as a first smooth curved surface representation, further smooth curved surface representations (ellipse cylinders) are fitted to the rib cage, by proceeding outwardly from the lung-based smooth curved surface representation. This can be done automatically by pattern recognition that identifies when an ellipse proceeds through a number of image points that represent a pair of ribs in the rib cage that exceeds a predetermined threshold, for example. Since the pairs of ribs in the rib cage will exhibit respectively different curvatures, more than one such smooth curved surface representation may be identified, as illustrated in the example shown in Figure 3 using ellipses as the smooth curved surface representations.

In step S4, once the one or more rib-based ellipses have been identified, the MR volume can be re-sampled based on cylinders associated therewith, by cutting and straightening the smooth curved surface representations, so that an image of the type shown in Figure 4 results. This image is then displayed in step S5.

Figure 6 is a block diagram showing the basic components of an apparatus for performing the inventive method. The apparatus includes a computer 4 with an input/output interface 5 that includes a display screen. The display screen can be an interactive screen, such as a touch-sensitive screen, that allows the aforementioned user interaction.

The computer 4 is in communication with a source 6 of an MR torso image. The source 6 may be an MR apparatus that includes an MR data acquisition scanner for acquiring such an image, or the source 6 may be a database in which a previously-acquired image has been entered and stored.

The computer 4 is provided with a non-transitory, computer readable data storage medium that is included with programming instructions that configure the computer 4 in order to perform the method according to the invention.

Although modifications and changes may be suggested by those skilled in the art, it is the intention of the Applicant to embody within the patent warranted hereon all changes and modifications as reasonably and properly come within the scope of the Applicant's contribution to the art.

## Claims

1. A method for rib unfolding from a magnetic resonance "MR" image, comprising the following computer-implemented method steps:
providing a computer (4) with an input data file (6) comprising volumetric MR data representing a 3D image of the rib cage and lungs of a subject;
selecting a view through said 3D image;
using said lungs in said 3D image to define a first smooth curved surface representation that is inside of said rib cage;
successively defining further smooth curved surface representations, starting from and moving outwardly from said first smooth curved surface representation by fitting further smooth curved surface representations to the rib cage, that each proceed through at least one pair of ribs in said rib cage;
unfolding said 3D image by cutting and straightening said further smooth curved surface representations, to obtain an unfolded MR image; and
at a display (7) in communication with said computer (4), displaying said unfolded MR image.

2. A method as claimed in claim 1 comprising identifying said lungs in said selected view by executing a lung segmentation algorithm in said computer (4).

3. A method as claimed in claim 2 comprising generating a segmentation mask in said segmentation algorithm, and generating a mesh of a surface of the lungs, and defining said further smooth curved surface representations by predicting said further smooth curved surface representations in said computer (4) using said lung surface.

4. A method as claimed in claim 3 comprising generating said mesh of said lung surface using an algorithm selected from the group consisting of marching cubes and marching tetrahedra.

5. A method as claimed in claim 1 comprising identifying said lungs in said selected view by displaying said selected view at a user interface and receiving a designation of said lungs into said computer (4) by manual interaction with said user interface that designates said lungs in said displayed selected view.

6. A method as claimed in claim 1 comprising, in said computer (4), defining each of said first smooth curved surface representation and said further smooth curved surface representations as a surface defined by an ellipse.

7. A method as claimed in claim 1 comprising, in said computer (4), defining each of said first smooth curved surface representation and said further smooth curved surface representations as a surface defined by a B-spline.

8. A method as claimed in claim 1 comprising, in said computer (4), defining each of said first smooth curved surface representation and said further smooth curved surface representations as a surface defined by a 3D Bezier curve.

9. An apparatus for rib unfolding from a magnetic resonance "MR" image, comprising:
a source of volumetric MR data representing a 3D image of the rib cage and lungs of a subject;
a computer (4) provided with an input data file (6) comprising said volumetric MR data;
said computer (4) being configured to carry out the following computer-implemented method steps:
selecting a view through said 3D image;
using said lungs in said 3D image to define a first smooth curved surface representation that is inside of said rib cage;
successively defining further smooth curved surface representations, starting from and moving outwardly from said first smooth curved surface representation by fitting further smooth curved surface representations to the rib cage, that each proceed through at least one pair of ribs in said rib cage;
unfolding said 3D image by cutting and straightening said further smooth curved surface representations, to obtain an unfolded MR image; and
a display (7) in communication with said computer (4), said computer (4) being configured to display said unfolded MR image at said display (7).

10. An apparatus as claimed in claim 9 wherein said computer (4) is configured to identify said lungs in said selected view by executing a lung segmentation algorithm in said computer (4).

11. An apparatus as claimed in claim 10 wherein said computer (4) is configured to generate a segmentation mask in said segmentation algorithm, and generating a mesh of a surface of the lungs, and defining said further smooth curved surface representations by predicting said further ellipses in said computer (4) using said lung surface.

12. An apparatus as claimed in claim 11 wherein said computer (4) is configured to generate said mesh of said lung surface using an algorithm selected from the group consisting of marching cubes and marching tetrahedra.

13. An apparatus as claimed in claim 9 wherein said computer (4) is configured to identify said lungs in said selected view by displaying said selected view at a user interface at said display (7) and to receive a designation of said lungs into said computer (4) by manual interaction with said user interface that designates said lungs in said displayed selected view.

14. An apparatus as claimed in claim 9 wherein said computer (4) is configured to define each of said first smooth curved surface representation and said further smooth curved surface representations as a surface defined by an ellipse.

15. An apparatus as claimed in claim 9 wherein said computer (4) is configured to define each of said first smooth curved surface representation and said further smooth curved surface representations as a surface defined by a B-spline.

16. An apparatus as claimed in claim 9 wherein said computer (4) is configured to define each of said first smooth curved surface representation and said further smooth curved surface representations as a surface defined by a 3D Bezier curve.

17. A non-transitory, computer-readable data storage medium encoded with programming instructions, said storage medium being loaded into a computer (4) and said programming instructions causing said computer (4) to:
receive with a computer (4) an input data file (6) comprising volumetric magnetic resonance "MR" data representing a 3D image of the rib cage and lungs of a subject;
select a view through said 3D image;
use said lungs in said 3D image to define a first smooth curved surface representation that is inside of said rib cage;
successively define further smooth curved surface representations, starting from and moving outwardly from said first smooth curved surface representation by fitting further smooth curved surface representations to the rib cage, that each proceed through at least one pair of ribs in said rib cage;
unfold said 3D image by cutting and straightening said further smooth curved surface representations, to obtain an unfolded MR image; and
at a display (7) in communication with said computer (4), display said unfolded MR image.

## Patentansprüche

1. Verfahren zur Rippenentfaltung aus einem Magnetresonanz, "MR",-Bild, umfassend die folgenden computerimplementierten Verfahrensschritte:
Versehen eines Computers (4) mit einer Eingabedatendatei (6), die MR-Volumendaten umfasst, die ein 3D-Bild des Brustkorbs und der Lungen eines Subjekts darstellen;
Auswählen einer Ansicht durch das 3D-Bild;
Verwenden der Lungen in dem 3D-Bild, um eine erste glatte, gekrümmte Oberflächendarstellung zu definieren, die im Innern des Brustkorbs liegt;
sukzessives Definieren weiterer glatter, gekrümmter Oberflächendarstellungen, ausgehend von der ersten glatten, gekrümmten Oberflächendarstellung und sich davon nach außen bewegend durch Anbringen weiterer glatter, gekrümmter Oberflächendarstellungen an dem Brustkorb, die jeweils durch mindestens ein Paar Rippen in dem Brustkorb durchgehen;
Entfalten des 3D-Bildes durch Schneiden und Geraderichten der weiteren glatten, gekrümmten Oberflächendarstellungen, um ein entfaltetes MR-Bild zu erhalten; und
auf einer Anzeige (7) in Verbindung mit dem Computer (4) Anzeigen des entfalteten MR-Bildes.

2. Verfahren nach Anspruch 1, umfassend das Identifizieren der Lungen in der ausgewählten Ansicht durch Ausführen eines Lungensegmentierungsalgorithmus in dem Computer (4).

3. Verfahren nach Anspruch 2, umfassend das Erzeugen einer Segmentierungsmaske in dem Segmentierungsalgorithmus und Erzeugen eines Netzes einer Oberfläche der Lungen und Definieren der weiteren glatten, gekrümmten Oberflächendarstellungen durch Vorhersagen der weiteren glatten, gekrümmten Oberflächendarstellungen in dem Computer (4) unter Verwendung der Lungenoberfläche.

4. Verfahren nach Anspruch 3, umfassend das Erzeugen des Netzes der Lungenoberfläche mithilfe eines Algorithmus, der aus der Gruppe bestehend aus wandernden Würfeln und wandernden Tetraedern ausgewählt ist.

5. Verfahren nach Anspruch 1, umfassend das Identifizieren der Lungen in der ausgewählten Ansicht durch Anzeigen der ausgewählten Ansicht auf einer Benutzeroberfläche und Empfangen einer Bezeichnung der Lungen in dem Computer (4) durch manuelle Interaktion mit der Benutzeroberfläche, die die Lungen in der angezeigten ausgewählten Ansicht bezeichnet.

6. Verfahren nach Anspruch 1, umfassend, in dem Computer (4), das Definieren von jeder der ersten glatten, gekrümmten Oberflächendarstellung und der weiteren glatten, gekrümmten Oberflächendarstellungen als eine Oberfläche, die von einer Ellipse definiert wird.

7. Verfahren nach Anspruch 1, umfassend, in dem Computer (4), das Definieren von jeder der ersten glatten, gekrümmten Oberflächendarstellung und der weiteren glatten, gekrümmten Oberflächendarstellungen als eine Oberfläche, die von einer B-Spline definiert wird.

8. Verfahren nach Anspruch 1, umfassend, in dem Computer (4), das Definieren von jeder der ersten glatten, gekrümmten Oberflächendarstellung und der weiteren glatten, gekrümmten Oberflächendarstellungen als eine Oberfläche, die von einer 3D-Bezier-Kurve definiert wird.

9. Vorrichtung zur Rippenentfaltung aus einem Magnetresonanz, "MR",-Bild, umfassend:
eine Quelle von MR-Volumendaten, die ein 3D-Bild des Brustkorbs und der Lungen eines Subjekts darstellen;
einen Computer (4), der mit einer Eingabedatendatei (6) versehen ist, die die MR-Volumendaten umfasst;
wobei der Computer (4) dazu ausgelegt ist, die folgenden computerimplementierten Verfahrensschritte auszuführen:
Auswählen einer Ansicht durch das 3D-Bild;
Verwenden der Lungen in dem 3D-Bild, um eine erste glatte, gekrümmte Oberflächendarstellung zu definieren, die im Innern des Brustkorbs liegt;
sukzessives Definieren weiterer glatter, gekrümmter Oberflächendarstellungen, ausgehend von der ersten glatten gekrümmten Oberflächendarstellung und sich davon nach außen bewegend durch Anbringen weiterer glatter, gekrümmter Oberflächendarstellungen an dem Brustkorb, die jeweils durch mindestens ein Paar Rippen in dem Brustkorb durchgehen;
Entfalten des 3D-Bildes durch Schneiden und Geraderichten der weiteren glatten, gekrümmten Oberflächendarstellungen, um ein entfaltetes MR-Bild zu erhalten; und
eine Anzeige (7) in Verbindung mit dem Computer (4), wobei der Computer (4) dazu ausgelegt ist, das entfaltete MR-Bild auf der Anzeige (7) anzuzeigen.

10. Vorrichtung nach Anspruch 9, wobei der Computer (4) dazu ausgelegt ist, die Lungen in der ausgewählten Ansicht durch Ausführen eines Lungensegmentierungsalgorithmus in dem Computer (4) zu identifizieren.

11. Vorrichtung nach Anspruch 10, wobei der Computer (4) dazu ausgelegt ist, eine Segmentierungsmaske in dem Segmentierungsalgorithmus zu erzeugen und ein Netz einer Oberfläche der Lungen zu erzeugen und die weiteren glatten, gekrümmten Oberflächendarstellungen durch Vorhersagen der weiteren glatten, gekrümmten Oberflächendarstellungen in dem Computer (4) unter Verwendung der Lungenoberfläche zu definieren.

12. Vorrichtung nach Anspruch 11, wobei der Computer (4) dazu ausgelegt ist, das Netz der Lungenoberfläche mithilfe eines Algorithmus, der aus der Gruppe bestehend aus wandernden Würfeln und wandernden Tetraedern ausgewählt ist, zu erzeugen.

13. Vorrichtung nach Anspruch 9, wobei der Computer (4) dazu ausgelegt ist, die Lungen in der ausgewählten Ansicht durch Anzeigen der ausgewählten Ansicht auf einer Benutzeroberfläche auf der Anzeige (7) zu identifizieren und eine Bezeichnung der Lungen in dem Computer (4) durch manuelle Interaktion mit der Benutzeroberfläche, die die Lungen in der angezeigten ausgewählten Ansicht bezeichnet, zu empfangen.

14. Vorrichtung nach Anspruch 9, wobei der Computer (4) dazu ausgelegt ist, jede der ersten glatten, gekrümmten Oberflächendarstellung und der weiteren glatten, gekrümmten Oberflächendarstellungen als eine Oberfläche zu definieren, die von einer Ellipse definiert wird.

15. Vorrichtung nach Anspruch 9, wobei der Computer (4) dazu ausgelegt ist, jede der ersten glatten, gekrümmten Oberflächendarstellung und der weiteren glatten, gekrümmten Oberflächendarstellungen als eine Oberfläche zu definieren, die von einer B-Spline definiert wird.

16. Vorrichtung nach Anspruch 9, wobei der Computer (4) dazu ausgelegt ist, jede der ersten glatten, gekrümmten Oberflächendarstellung und der weiteren glatten, gekrümmten Oberflächendarstellungen als eine Oberfläche zu definieren, die von einer 3D-Bezier-Kurve definiert wird.

17. Nichtflüchtiges computerlesbares Datenspeichermedium, das mit Programmieranweisungen kodiert ist, wobei das Speichermedium in einen Computer (4) geladen wird und die Programmierungsanweisungen bewirken, dass der Computer (4):
mit einem Computer (4) eine Eingabedatendatei (6) empfängt, die Magnetresonanz, "MR",-Volumendaten umfasst, die ein 3D-Bild des Brustkorbs und der Lungen eines Subjekts darstellen;
eine Ansicht durch das 3D-Bild auswählt;
die Lungen in dem 3D-Bild verwendet, um eine erste glatte, gekrümmte Oberflächendarstellung zu definieren, die im Innern des Brustkorbs liegt;
sukzessive weitere glatte, gekrümmte Oberflächendarstellungen definiert, ausgehend von der ersten glatten, gekrümmten Oberflächendarstellung und sich davon nach außen bewegend durch Anbringen weiterer glatter, gekrümmter Oberflächendarstellungen an dem Brustkorb, die jeweils durch mindestens ein Paar Rippen in dem Brustkorb durchgehen;
das 3D-Bild durch Schneiden und Geraderichten der weiteren glatten, gekrümmten Oberflächendarstellungen entfaltet, um ein entfaltetes MR-Bild zu erhalten; und
auf einer Anzeige (7) in Verbindung mit dem Computer (4) das entfaltete MR-Bild anzeigt.

## Revendications

1. Procédé de déploiement de côtes à partir d'une image de résonance magnétique « RM », comprenant les stades de procédé suivants mis en œuvre par ordinateur :
on se procure un ordinateur (4) ayant un fichier (6) de données d'entrée, comprenant des données volumétriques RM représentant une image en 3D de la cage thoracique et des poumons d'un sujet ;
on sélectionne une vue dans l'image en 3D ;
on utilise les poumons dans l'image en 3D pour définir une première représentation de surface courbée de manière régulière, qui est à l'intérieur de la cage thoracique ;
on définit successivement d'autres représentations de surface courbées de manière régulière en partant et en se déplaçant vers l'extérieur à partir de la première représentation de surface courbée de manière régulière, en ajustant d'autres représentations de surface courbées de manière régulière à la cage thoracique, de manière à ce que chacune avance dans au moins une paire de côtes de la cage thoracique ;
on déploie l'image en 3D en coupant et en redressant les autres représentations de surface courbées de manière régulière pour obtenir une image déployée RM ; et
à un affichage (7), en communication avec l'ordinateur (4), on affiche l'image déployée RM.

2. Procédé suivant la revendication 1, comprenant l'identification des poumons dans la vue sélectionnée, en exécutant un algorithme de segmentation des poumons dans l'ordinateur (4).

3. Procédé suivant la revendication 2, comprenant la création d'un masque de segmentation dans l'algorithme de segmentation et la création d'une maille d'une surface des poumons et la définition des autres représentations de surface courbées de manière régulière en prédisant les autres représentations de surface courbées de manière régulière dans l'ordinateur (4), en utilisant la surface des poumons.

4. Procédé suivant la revendication 3, comprenant la production de la maille de la surface des poumons, en utilisant un algorithme choisi dans le groupe consistant en des cubes de marche et des tétraèdres de marche.

5. Procédé suivant la revendication 1, comprenant l'identification des poumons dans la vue sélectionnée, en affichant la vue sélectionnée à une interface d'utilisateur et en recevant une désignation des poumons dans l'ordinateur (4) par interaction manuelle avec l'interface d'utilisateur, qui désigne les poumons dans la vue sélectionnée affichée.

6. Procédé suivant la revendication 1, comprenant, dans l'ordinateur (4), la définition de chacune de la première représentation de surface courbée de manière régulière et des autres représentations de surface courbées de manière régulière, comme une surface définie par une ellipse.

7. Procédé suivant la revendication 1, comprenant, dans l'ordinateur (4), la définition de chaque première représentation de surface courbée de manière régulière et des autres représentations de surface courbées de manière régulière, comme une surface définie par un spline B.

8. Procédé suivant la revendication 1, comprenant, dans l'ordinateur (4), la définition de chacune de la première représentation de surface courbée de manière régulière et les autres représentations de surface courbées de manière régulière, comme une surface définie par une courbe de Bézier en 3D.

9. Dispositif pour déployer des côtes à partir d'une image de résonnance magnétique « RM », comprenant :
une source de données volumétriques RM représentant une image en 3D de la cage thoracique et des poumons d'un sujet ;
un ordinateur (4) pourvu d'un fichier (6) de données d'entrée, comprenant les données volumétriques RM ;
l'ordinateur (4) étant configuré pour effectuer les stades de procédé suivants mis en œuvre par ordinateur :
sélectionner une vue dans l'image en 3D ;
utiliser les poumons dans l'image en 3D pour définir une première représentation de surface courbée de manière régulière, qui est à l'intérieur de la cage thoracique ;
définir successivement d'autres représentations de surface courbées de manière régulière en partant et en se déplaçant vers l'extérieur à partir de la première représentation de surface courbée de manière régulière, en ajustant d'autres représentations de surface courbées de manière régulière à la cage thoracique, de manière à ce que chacune avance dans au moins une paire de côtes de la cage thoracique ;
déployer l'image en 3D en coupant et en redressant les autres représentations de surface courbées de manière régulière pour obtenir une image déployée RM ; et
à un affichage (7), en communication avec l'ordinateur (4), afficher l'image déployée RM.

10. Dispositif suivant la revendication 9, dans lequel l'ordinateur (4) est configuré pour identifier les poumons dans la vue sélectionnée, en exécutant un algorithme de segmentation des poumons dans l'ordinateur (4).

11. Dispositif suivant la revendication 10, dans lequel l'ordinateur (4) est configuré pour créer un masque de segmentation dans l'algorithme de segmentation et pour créer une maille d'une surface des poumons et pour définir les autres représentations de surface courbées de manière régulière, en prédisant d'autres ellipses dans l'ordinateur (4), en utilisant la surface des poumons.

12. Dispositif suivant la revendication 11, dans lequel l'ordinateur (4) est configuré pour créer une maille de la surface des poumons, en utilisant un algorithme choisi dans le groupe consistant en des cubes de marche et des tétraèdres de marche.

13. Dispositif suivant la revendication 9, dans lequel l'ordinateur (4) est configuré pour identifier les poumons dans la vue sélectionnée, en affichant la vue sélectionnée à une interface d'utilisateur et en recevant une désignation des poumons dans l'ordinateur (4) par interaction manuelle avec l'interface d'utilisateur, qui désigne les poumons dans la vue sélectionnée affichée.

14. Dispositif suivant la revendication 9, dans lequel l'ordinateur (4) est configuré pour définir chacune de la première représentation de surface courbée de manière régulière et des autres représentations de surface courbées de manière régulière, comme une surface définie par une ellipse.

15. Dispositif suivant la revendication 9, dans lequel l'ordinateur (4) est configuré pour définir chaque première représentation de surface courbée de manière régulière et des autres représentations de surface courbées de manière régulière, comme une surface définie par un spline B.

16. Dispositif suivant la revendication 9, dans lequel l'ordinateur (4) est configuré pour définir chacune de la première représentation de surface courbée de manière régulière et les autres représentations de surface courbées de manière régulière, comme une surface définie par une courbe de Bézier en 3D.

17. Support de mémoire de données non transitoire, déchiffrable par ordinateur et codé par des instructions de programmation, le support de mémoire étant chargé dans un ordinateur (4) et les instructions de programmation faisant que l'ordinateur (4) :
reçoit, par un ordinateur (4), un fichier (6) de données d'entrée, comprenant des données volumétriques de résonance magnétique « RM » représentant une image en 3D de la cage thoracique et des poumons d'un sujet ;
sélectionne une vue dans l'image en 3D ;
utilise les poumons dans l'image en 3D pour définir une première représentation de surface courbée de manière régulière, qui est à l'intérieur de la cage thoracique ;
définit successivement d'autres représentations de surface courbées de manière régulière en partant et en se déplaçant vers l'extérieur à partir de la première représentation de surface courbée de manière régulière, en ajustant d'autres représentations de surface courbées de manière régulière à la cage thoracique, de manière à ce que chacune avance dans au moins une paire de côtes de la cage thoracique ;
déploie l'image en 3D en coupant et en redressant les autres représentations de surface courbées de manière régulière pour obtenir une image déployée RM ; et
à un affichage (7), en communication avec l'ordinateur (4), affiche l'image déployée RM.
